# EUROPÄISCHE PATENTSCHRIFT

(11) **EP 1 579 887 B1**
(45) Veröffentlichungstag und Bekanntmachung des Hinweises auf die Patenterteilung: **19.06.2013**
(21) Anmeldenummer: 05006111.8
(22) Anmeldetag: 21.03.2005
(51) Int. Cl.: A61M 5/165, A61M 39/16

(54) **Verbindungsvorrichtung für einen Katheter, insbesondere für einen zentralen Venenkatheter**
Connector for a catheter, especially a central venous catheter
Connecteur pour un cathéter, en particulier pour cathéter veneux central

(30) Priorität: 24.03.2004 DE 102004014874
(43) Veröffentlichungstag der Anmeldung: 28.09.2005
(73) Patentinhaber: Reichardt, André, 52222 Stolberg (DE)
(72) Erfinder: Reichardt, André, 52222 Stolberg (DE)
(74) Vertreter: Naeven, Ralf

(56) Entgegenhaltungen:
- EP-A- 0 441 171
- DE-U1- 9 320 337
- US-A- 4 309 992
- US-A- 5 049 139
- US-A- 5 782 808

## Beschreibung

Die Erfindung betrifft eine Verbindungsvorrichtung für einen Katheter, insbesondere für einen zentralen Venenkatheter, mit einem Einlaufanschlussstück, einem Auslaufanschlussstück, einem zwischen Einlaufanschlussstück und Auslaufanschlussstück angeordneten Zwischenstück und einem Infusionskanal, wobei mindestens ein den Durchfluss von Infusionsflüssigkeit erlaubendes und gleichzeitig den Bakterien- und/oder Pilzbefall des Katheters hemmendes Schutzelement aus Silber vorgesehen ist.

Eine Verbindungsvorrichtung der vorgenannten Art ist aus US 4,309,992 bekannt. Darin ist ein antibiotischer Filter offenbart, der z. B. bei der peritonealen Dialyse zwischen der die Haut des Patienten penetrierenden Kanüle und dem Reservoir für die Dialyse-Lösung eingesetzt werden kann. Der antibakterielle Filter besteht aus einem Gehäuse, das mit kugelförmigen Filterelementen aus Silber gefüllt ist. Dabei ist der gesamte Infusionskanal zwischen zwei porösen Grenzplatten mit den Silberkugeln ausgefüllt. Da die Infusionsflüssigkeit durch den Silberkugel-Filter gezogen wird, entfaltet das Silber seine bekannte keimhemmende Wirkung für die Infusionsflüssigkeit. Wegen der Vielzahl der einzusetzenden Kugeln ist eine Auswechslung allein des Schutzelements aus Silber und eine Reinigung desselben aufwendig.

Aus der US 5,049,139 ist eine Verbindungsvorrichtung bekannt, die für den Einsatz bei einem Katheter geeignet ist und bei der ebenfalls die antibakterielle Wirkung von Silberionen genutzt wird. Hierfür wird in der Verbindungsvorrichtung eine Hülse aus einem wasserlöslichen Glas verwendet, das mit Silber imprägniert oder versetzt ist. Das Glas löst sich allmählich auf und setzt dadurch das Silber mit seinen antibakteriellen Eigenschaften über eine längere Periode frei. Dabei kann über die Zusammensetzung des Glases die Silber-Abgaberate festgelegt werden.

Des Weiteren ist eine für einen Katheter geeignete Verbindungsvorrichtung aus der DE 101 46 853 A1 bekannt. Dort ist eine Verweilkatheteranordnung offenbart mit einem flexiblen Katheter, durch den beim Legen des Katheters ein Führungselement verläuft.

In Flussrichtung vor dem Katheter ist eine Verbindungsvorrichtung angeordnet, an dessen Einlaufanschlussstück ein Infusionsschlauch und an das Auslaufanschlussstück eine Halterung für Katheter und Führungselement angeschlossen werden kann. In der Verbindungsvorrichtung ist ein Bakterienfilter vorgesehen, der die Verbindungsvorrichtung vollständig ausfüllt und nicht auswechselbar zu sein scheint.

Eine Verbindungsvorrichtung für den wechselnden Anschluss, z. B. von Infusionsschläuchen an einen Katheter ohne Bakterienfilter ist aus der DE 300 090 3 C1 bekannt.

Aus der US 5,782,808 ist eine antibakteriell wirkende Verbindungsvorrichtung bekannt, bei der die Oberfläche des Infusionskanals mit Silber beschichtet ist.

Insbesondere bei zentralen Venenkathetem, die über lange Zeiträume über eine künstliche offene Wunde im Körper verbleiben, z. B. zur künstlichen Ernährung bei Kurzdarmsyndrom, ist die Gefahr von in der Regel durch den Eintritt von Bakterien verursachten Sepsen recht groß, da absolut sterile Bedingungen kaum geschaffen werden können. Zudem sind auch fehlerhafte Handhabungen nicht auszuschließen, die zum Bakterienbefall führen. Derartige Sepsen sind für die behandelte Person mit erheblich persönlichen Nachteilen verbunden. Hinzu bringt jede Sepsis erhebliche Mehrkosten mit sich.

Nach Beendigung einer Infusion wird das Endstück des Katheters, oder - falls ein Verbindungsstück vorhanden ist - das distale Ende des Verbindungsstücks gereinigt, der Katheter mit Lösung gefüllt und am Endstück beziehungsweise am distalen Ende des Verbindungsstücks mit einem Stopfen verschlossen. Trotz Vorsichtsmaßnahmen, wie Spülung, Handschuhe für das bedienende Personal etc. können sich Bakterien und/oder Pilze im Endbereich des Katheters oder des Verbindungsstücks absetzen. Diese werden trotz Spülung oftmals nicht vollständig entfernt. Im Zeitraum zwischen zwei Infusionen können sich derartige Bakterien oder Pilze im Endstück beziehungsweise im Verbindungsstück stark vermehren. Von dort werden sie mit der nächsten Infusion in den Körper des Patienten gespült und können Sepsen hervorrufen.

Der Erfindung liegt die Aufgabe zugrunde, auf alternative Weise das Eindringen von Bakterien und/oder Pilzen in den Katheter zu verhindern oder zumindest erheblich zu reduzieren.

Erfindungsgemäß wird diese Aufgabe bei einer Verbindungsvorrichtung der eingangs genannten Art dadurch gelöst, dass das Schutzelement ein im Infusionskanal oder in Infusionsflussrichtung gesehen am Ende des Infusionskanals angeordnetes, zahlreiche Öffnungen aufweisendes Siebelement aus Silber ist.

Das Schutzelement stellt eine Barriere für Bakterien und/oder Pilze dar. Die Anordnung dieses Schutzelements in der Verbindungsvorrichtung ist insbesondere deshalb vorteilhaft, weil die komplette Verbindungsvorrichtung zusammen mit dem Schutzelement auf sehr einfache Art und Weise ausgetauscht werden kann, wenn das Schutzelement z. B. in Folge einer Verstopfung, unwirksam werden sollte. Derartige Schutzelemente in Infusionsflussrichtung gesehen hinter der Verbindungsvorrichtung, also im Katheter selbst, hätten demgegenüber den Nachteil, dass der gesamte Katheter ausgewechselt werden müsste. Ein Schutzelement in Infusionsflussrichtung gesehen vor der Verbindungsvorrichtung brächte keine Vorteile im Hinblick auf die Risiken, die beim Wechseln der Infusionsschläuche auftreten. Es ist davon auszugehen, dass das Verbindungsstück wesentlich seltener gewechselt werden muss, als die Infusionsschläuche, so dass gegenüber dem Stand der Technik eine entsprechende Reduktion des Sepsis-Risikos erreicht werden kann.

Es ist seit langem bekannt, dass Silber eine keimhemmende Wirkung hat. Das Siebelement aus Silber stellt somit ein wirksames Hindernis für Keime dar, die - insbesondere beim Wechsel des Infusionsschlauches - in die Verbindungsvorrichtung gelangt sind. Das Siebelement kann zudem gereinigt, desinfiziert und wieder verwendet werden.

Des Weiteren kann die erfindungsgemäße Verbindungsvorrichtung so ausgebildet sein, dass das Siebelement eine im Wesentlichen kegelstumpfförmige Mantelfläche aufweist und die Öffnungen die Mantelfläche durchsetzen.

Auf diese Weise wird eine relativ große Oberfläche des Siebelements erreicht, die entsprechend vorteilhaft für die gewollte Keim hemmende Wirkung ist.

Weiterhin kann die erfindungsgemäße Verbindungsvorrichtung so ausgebildet sein, dass zusätzlich zum und in Infusionsflussrichtung gesehen vor dem Siebelement im Zwischenstück ein Bakterienfilter angeordnet ist.

Es können auch Anwendungen vorteilhaft sein, bei denen ohne Bakterienfilter und allein mit dem Siebelement aus Silber gearbeitet wird.

Schließlich kann die erfindungsgemäße Verbindungsvorrichtung so ausgebildet sein, dass der Infusionskanal zumindest zum Teil an der Oberfläche Silber aufweist. Dies kann z. B. durch eine Beschichtung erreicht werden oder indem der Infusionskanal oder die gesamte Verbindungsvorrichtung aus Silber besteht.

Im Folgenden wird eine mögliche Ausbildungsform der erfindungsgemäßen Verbindungsvorrichtung anhand einer Figur dargestellt.

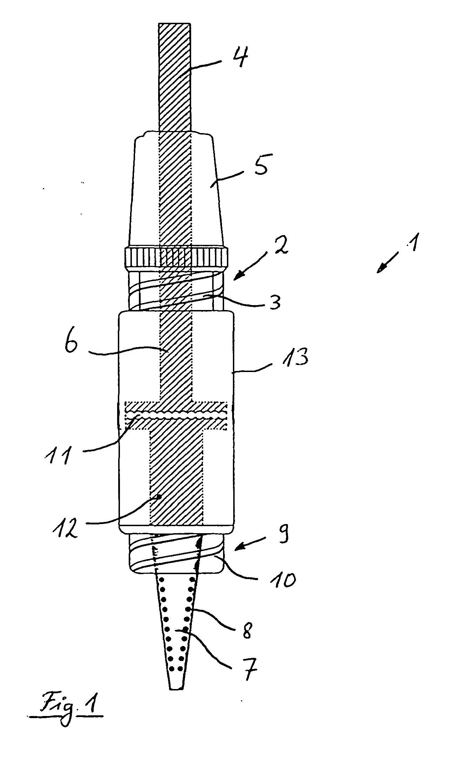

Die einzige Figur zeigt eine Verbindungsvorrichtung 1 mit einem Einlaufanschlussstück 2 mit einem Gewinde 3, über das ein Infusionsschlauch 4 mittels eines Schlauchanschlussstückes 5 angeschlossen werden kann. Alternativ zum Infusionsschlauch 4 kann auch ein Mehrwegeelement angeschlossen werden, an das wiederum mehrere Zuläufe, z. B. für die Infusionsflüssigkeit und für Medikamente, anschließbar sind. Ein angeschlossener Infusionsschlauch 4 endet an einer hier nicht gesondert dargestellten Übergangsstelle in einen Infusionskanal 6 der Verbindungsvorrichtung 1. Der Infusionskanal 6 durchläuft ein Zwischenstück 13 und endet in einem siebartigen Infusionsauslauf 7 aus Silber, der auf seiner kegelstumpfförmigen Mantelfläche zahlreiche Öffnungen 8 aufweist, durch die die im Infusionskanal 6 befindliche Infusionsflüssigkeit austreten kann. Der Infusionsauslauf 7 ragt über das Auslaufanschlussstück 9 hinaus, das ebenfalls ein Gewinde 10 zum Anschluss an einen hier nicht dargestellten Katheter aufweist.

Im Infusionskanal 6 ist ein Bakterienfilter 11 angeordnet. Der Bakterienfilter 11 befindet sich in einem aufgeweiteten Bereich des Infusionskanals 6. Aufgrund dieser Erweiterung kann eine größere Oberfläche des Bakterienfilters 11 wirksam werden und stellt somit für den Durchfluss der Infusionsflüssigkeit ein geringeres Hindemis dar. Zwischen Bakterienfilter 11 und Auslaufanschlussstück 9 ist ein Luftaustrittsventil 12 vorgesehen, durch das eventuell auftretende gasförmige Einschlüsse austreten können.

### Bezugszeichenliste

- 1: Verbindungsvorrichtung
- 2: Einlaufanschlussstück
- 3: Gewinde
- 4: Infusionsschlauch
- 5: Schlauchanschlussstück
- 6: Siebelement
- 7: Infusionsauslauf
- 8: Öffnung
- 9: Auslaufanschlussstück
- 10: Gewinde
- 11: Bakterienfilter
- 12: Luftaustrittsventil
- 13: Zwischenstück

## Patentansprüche

1. Verbindungsvorrichtung für einen Katheter, insbesondere für einen zentralen Venenkatheter, mit einem Einlaufanschlussstück (2), einem Auslaufanschlussstück (9), einem zwischen Einlaufanschlussstück (2) und Auslaufanschlussstück (9) angeordneten Zwischenstück (13) und einem Infusionskanal (6),
wobei mindestens ein den Durchfluss von Infusionsflüssigkeit erlaubendes und gleichzeitig den Bakterien- und/oder Pilzbefall des Katheters hemmendes Schutzelement aus Silber vorgesehen ist,
**dadurch gekennzeichnet,**
**dass** das Schutzelement ein im Infusionskanal (6) oder in Infusionsflussrichtung gesehen am Ende des Infusionskanals (6) angeordnetes, zahlreiche Öffnungen (8) aufweisendes Siebelement (7) aus Silber ist.

2. Verbindungsvorrichtung nach Anspruch 1, **dadurch gekennzeichnet, dass** das Siebelement (7) eine im Wesentlichen kegelstumpfförmige Mantelfläche aufweist und die Öffnungen (8) die Mantelfläche durchsetzen.

3. Verbindungsvorrichtung nach Anspruch 1 oder 2, **dadurch gekennzeichnet, dass** zusätzlich zum und in Infusionsflussrichtung gesehen vor dem Siebelement (7) im Zwischenstück (13) ein Bakterienfilter (11) angeordnet ist.

4. Verbindungsvorrichtung nach einem der Ansprüche 1 bis 3, **dadurch gekennzeichnet, dass** der Infusionskanal (6) zumindest zum Teil an der Oberfläche Silber aufweist.

## Claims

1. Connector for a catheter, especially for a central venous catheter, with an inlet attachment piece (2), an outlet attachment piece (9), an intermediate piece (13) arranged between inlet attachment piece (2) and outlet attachment piece (9), and an infusion channel (6), wherein at least one protective element made of silver is provided which allows infusion liquid to flow through and which at the same time inhibits the bacterial and/or fungal infestation of the catheter, **characterized in that** the protective element is a sieve element (7) which is made of silver, has numerous openings (8) and is arranged in the infusion channel (6) or, as seen in the infusion flow direction, at the end of the infusion channel (6).

2. Connector according to Claim 1, **characterized in that** the sieve element (7) has a substantially frustoconical jacket surface, and the openings (8) extend through the jacket surface.

3. Connector according to Claim 1 or 2, **characterized in that** a bacteria filter (11) is arranged in the intermediate piece (13) in addition to and, as seen in the infusion flow direction, upstream of the sieve element (7).

4. Connector according to one of Claims 1 to 3, **characterized in that** the infusion channel (6) has silver on at least part of the surface.

## Revendications

1. Dispositif de connexion pour un cathéter, en particulier pour un cathéter veineux central, comprenant un élément de raccordement d'entrée (2), un élément de raccordement de sortie (9), un élément intermédiaire (13) disposé entre l'élément de raccordement d'entrée (2) et l'élément de raccordement de sortie (9) et un canal de perfusion (6), au moins un élément de protection en argent permettant le passage du liquide de perfusion et empêchant simultanément la infestation bactérienne et/ou fongique du cathéter,
**caractérisé en ce que**
l'élément de protection est un élément de tamis (7) en argent disposé dans le canal de perfusion (6) ou, vu dans la direction du flux de perfusion, à l'extrémité du canal de perfusion (6), et présentant une pluralité d'ouvertures (8).

2. Dispositif de connexion selon la revendication 1, **caractérisé en ce que** l'élément de filtre (7) présente une surface d'enveloppe de forme essentiellement tronconique et les ouvertures (8) traversent la surface d'enveloppe.

3. Dispositif de connexion selon la revendication 1 ou 2, **caractérisé en ce qu'**un filtre à bactéries (11) est disposé en plus de l'élément de tamis (7) et, vu dans la direction du flux de perfusion, avant l'élément filtrant (7), dans l'élément intermédiaire (13).

4. Dispositif de connexion selon l'une quelconque des revendications 1 à 3, **caractérisé en ce que** le canal de perfusion (6) présente de l'argent au moins en partie sur la surface.
